# EUROPEAN PATENT SPECIFICATION

(11) **EP 2 797 583 B1**
(45) Date of publication and mention of the grant of the patent: **09.09.2015**
(21) Application number: 12826586.5
(22) Date of filing: 25.12.2012
(51) Int. Cl.: A61K 9/00, A61K 31/00, A61K 47/00

(54) **COMBINED PHARMACEUTICAL FORMULATION CONTAINING DIACEREIN**
KOMBINIERTE PHARMAZEUTISCHE FORMULIERUNG MIT DIACEREIN
FORMULATION PHARMACEUTIQUE COMBINÉE CONTENANT DE LA DIACÉRÉINE

(30) Priority: 27.12.2011 TR 201113006; 01.08.2012 TR 201208987
(43) Date of publication of application: 05.11.2014
(73) Proprietor: Sanovel Ilac Sanayi Ve Ticaret Anonim Sirketi, 34460 Istanbul (TR)
(72) Inventor: CIFTER, Umit, 34460 Istanbul (TR); TURKYILMAZ, Ali, 34460 Istanbul (TR); MUTLU, Onur, 34460 Istanbul (TR)
(74) Representative: Sevinç, Erkan
(86) International application number: PCT/TR2012/000246
(87) International publication number: WO 2013/100881

(56) References cited:
- EP-A2- 0 371 431
- GB-A- 2 432 526
- US-A- 4 996 209
- US-A1- 2004 039 366

## Description

### Technical Field

The present invention relates to a new solid oral dosage form comprising a pharmaceutically effective amount of NSAID and a pharmaceutically effective amount of diacerein and the solid oral dosage form according to the present invention is preferably in the form of tablet and more preferably in the form of multi-layer tablet.

### Background of the invention

Non-steroidal anti-inflammatory drugs (NSAIDs) are drugs with analgesic, anti-inflammatory, antipyretic and osteoarthritis therapeutic activities and therefore are frequently used. NSAIDs are well defined group of compounds and comprise molecules derivatives of salicylate, acetic acid, propionic acid. Propionic acid derivative NSAIDs can be given as ibuprofen, naproxen, ketoprofen, fenoprofen, benoxaprofen, suprofen, flurbiprofen, ibuproxam, alminoprofen, dexibuprofen, dexketoprofen and indoprofen. These molecules are used in the treatment or prevention of symptoms such as pain, inflammation and fever. These symptoms also include those of musculoskeletal and joint disorders such as ankylosing spondylitis, osteoarthritis and rheumatoid arthritis, soft tissue injuries such as sprains and strains, post-operative pain, dolorous and difficult menstruation and migraine pains.

Another molecule that can be used especially in the treatment of osteoarthritis is diacerein disclosed in the patent US4244968A. Said molecule is interleukin-1 inhibitor having anthraquinone structure and chemical structure thereof is shown in Formula 1.

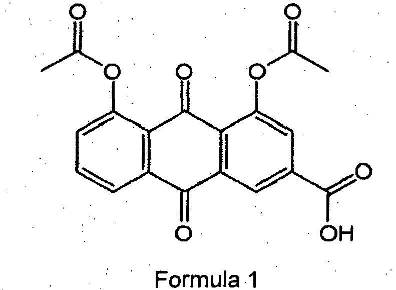

Use of NSAIDs and diacerein may cause some, particularly gastrointestinal, side effects. The most common side effect of NSAIDs is defined as a burning sensation in the gastrointestinal system. Diacerein, on the other hand, shows gastrointestinal side effect of diarrhea. Avoiding systemic adverse effects of NSAIDs and diacerein is very important for the patient. To prevent these side effects, various coating processes and formulations with different release profiles have been developed. For example, flurbiprofen is enterically coated in order to minimize damage to the stomach (Hashmat D., Shoaib H., Mehmood Z. (2008) AAPS PharmaSci Tech 9(1): 116-121) or formulated so as to provide controlled release (EP0234670). Furthermore, as described in the patent numbered EP1169032B1, ketoprofen, which is another propionic acid derivative, is coated with a polymeric layer providing sustained release. Side effects of diacerein is tried to be minimized by formulating it in hydrogel matrix (EP0809995). NSAIDs and diacerein are molecules with poor solubility in water. If a molecule has poor water solubility; absorption and hence bioavailability of said molecule are low. Thus, slow and late release of NSAIDs and diacerein, having already poor solubility, from the formulations developed in the prior art decreases the bioavailability of these two molecules.

Diacerein is available in the market under the brand names such as Artrodar®, Rexena® and is in the form of capsule. As is known, diacerein has a different mechanism of action than the other NSAIDs, however the use thereof with other NSAIDs can cause an additional effect. For this reason, today, although an oral pharmaceutical formulation comprising combination of diacerein and NSAID doesn't exist, these two molecules are used together, especially in the treatment of osteoarthritis. However, caution must be taken when using said two molecules simultaneously considering the side effects that can be caused by them. In addition, lack of a formulation comprising the two molecules together causes the patients to carry more than one drug and difficulty in administration of said drugs. Apart from that, another drawback is the intake of excipients in excess amount resulting from the intake of more than one drug, because excipients can also cause side effects as the active ingredients.

There are many points to be considered while formulating two active ingredients in a pharmaceutical formulation. These active ingredients should not be adversely affected from each other, at the same time, the two active ingredients should be released from the formulation in a concordant and coordinated manner. In addition, NSAIDs and diacerein, as in the case of many active ingredients, show stability problems resulting from the influence of the environmental and physical conditions. They are affected greatly by the temperature, light, air and humidity conditions; additionally, they are also affected by the solvents used during formulation thereof. Exposure to air and moisture causes structural destruction of said active ingredients and leads to the development of chemical behavior change. The stability of the developed products is not at a desired level and the shelf life thereof is shortened.

Consequently, stable pharmaceutical formulations with therapeutic analgesic, antipyretic and osteoarthritis activities, where NSAID and diacerein can be formulated together are still needed.

### Objects of the Invention

The object of the present invention is to obtain a combined pharmaceutical formulation comprising an NSAID and diacerein, having therapeutic analgesic, antipyretic and/or osteoarthritis activities, eliminating all the aforementioned drawbacks and providing additional advantages to the respective technical field.

Another object of the present invention is to increase the effectiveness of the patient's diacerein treatment by administering NSAID to the patient being treated continuously with diacerein.

Another object of the present invention is to obtain a pharmaceutical formulation providing anti-inflammatory, analgesic, antipyretic and/or osteoarthritis treatment wherein NSAID and diacerein molecules do not adversely affect each other physically and chemically.

Another object of the present invention is to obtain a pharmaceutical formulation comprising a pharmaceutically effective amount of NSAID and a pharmaceutically effective amount of diacerein with desired level of solubility and dissolution rate of said two molecules, thus, with desired level of bioavailability and being released quickly.

Another object of the present invention is to obtain a combined pharmaceutical formulation comprising a pharmaceutically effective amount of NSAID and a pharmaceutically effective amount of diacerein with said two molecules being released in a coordinated manner.

Another object of the present invention is to obtain a pharmaceutical formulation comprising an NSAID and diacerein, having therapeutic anti-inflammatory analgesic, antipyretic and/or osteoarthritis activities with gastrointestinal side effects being minimized.

Another object of the present invention is to decrease, by means of a unit dosage form comprising NSAID and diacerein, the amount of excipients (e.g., lactose) that could especially cause side effects when said molecules are administered individually.

Another object of the present invention is to obtain a stable unit dosage form comprising a pharmaceutically effective amount of NSAID together with a pharmaceutically effective amount of diacerein and at least one pharmaceutically acceptable excipient. Accordingly, the object of the present invention is to select the suitable excipient composition for providing the stability within the tablet.

### Description of the Figures

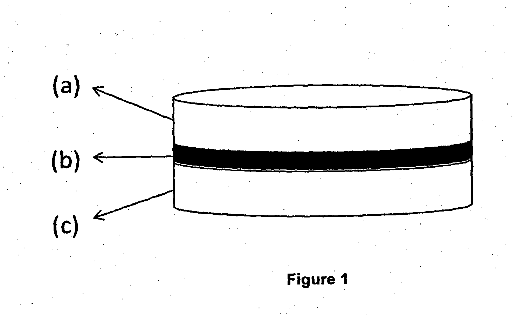

**Figure 1** shows a solid multi-layer oral tablet formulation comprising the following: i) a first layer containing the first active agent (a); ii) a second layer containing the second active agent (c); and iii) a barrier layer separating said two layers (b).

### Detailed Description of the Invention

The pharmaceutical formulation according to the present invention is effective in prevention and reduction and/or treatment of symptoms such as pain, inflammation and/or fever and osteoarthritis wherein said composition comprises combination of an NSAID and diacerein. These symptoms also include those of musculoskeletal and joint disorders such as ankylosing spondylitis, osteoarthritis and rheumatoid arthritis, soft tissue injuries such as sprains and strains, post-operative pain, dolorous and difficult menstruation migraine pains and all forms of headaches.

The present invention relates to a new solid oral dosage form comprising a pharmaceutically effective amount of NSAID and a pharmaceutically effective amount of diacerein. The solid oral dosage form according to the present invention is preferably in the form of tablet and more preferably in the form of multi-layer tablet.

The present invention relates to a solid oral multi-layer tablet formulation comprising the followings: i) a first layer containing an NSAID molecule as the first active agent and pharmaceutically acceptable excipient or excipients; ii) a second layer containing diacerein molecule as the second active agent and pharmaceutically acceptable excipient or excipients; and iii) a barrier layer positioned between said two layers and containing pharmaceutically acceptable excipient or excipients (Figure 1). In the tablet, these 3 layers are provided in the form of sandwich. Barrier layer is provided in the intermediate portion so as to separate NSAID and diacerein layers from each other and NSAID and diacerein layers are provided on both sides of the barrier layer on the outside portion. Negative interaction of NSAID and diacerein molecules are prevented by means of the barrier layer provided in the intermediate portion.

The NSAID in the multi-layer tablet formulation according to the present invention is a propionic acid derivative wherein it can be given as ibuprofen, naproxen, ketoprofen, fenoprofen, benoxaprofen, suprofen, flurbiprofen, ibuproxam, alminoprofen, dexibuprofen, dexketoprofen, indoprofen and mixture thereof and preferably said NSAID is flurbiprofen, dexketoprofen and mixture thereof.

On the other hand, NSAID and diacerein in the multi-layer tablet formulation according to the present invention can be present as pharmaceutically acceptable salts, enantiomers, racemates, polymorphs, esters and/or hydrates thereof.

NSAID group molecules and diacerein are molecules wherein its absorption occurs in the gastrointestinal system. The multi-layer tablet according to the present invention, as per tablet shape, provides disintegration of both molecule layers when taken into the body. NSAID and diacerein layers starts to disintegrate when taken into the body as it doesn't contain any coating or top layer. Resulting from said disintegration, NSAID and diacerein molecules solubilizes. Thus, formulation of said molecules having very low solubility in the multi-layer tablet according to the present invention positively affects the dissolution profiles of said molecules.

Another positive effect of the multi-layer tablet formulation according to the present invention is the prevention of adverse physical and chemical interaction of NSAID and diacerein molecules, provided in two separate layers and thus, a more stable formulation is provided.

The pharmaceutically acceptable excipients provided in the multi-layer tablet formulation according to the present invention can be selected from, but not limited to, diluents, binders, disintegrants, glidants, lubricants, plasticizers, surfactants, preservatives or mixtures thereof.

Diluents according to the present invention include, but not limited to, lactose, microcrystalline cellulose, corn starch, modified corn starch, calcium phosphate, sugar, dextrose, mannitol, sorbitol, starch, pregelatinized starch and mixtures thereof.

Binders according to the present invention include, but not limited to, lactose, polymethacrylate, polyvinylpyrrolidone (povidone), hydroxypropyl methyl cellulose (HPMC), hydroxypropyl cellulose (HPC), carboxymethyl cellulose (CMC), methyl cellulose (MC), hydroxyethyl cellulose, sodium carboxy methyl cellulose (NaCMC), carboxymethyl cellulose calcium, ethyl cellulose, polyethylene oxide, gelatin, starch, pregelatinized starch, xanthan gum, guar gum, alginate, carrageenan, pectin, carbomer, cellulose acetate phthalate, hydroxy propyl starch, polaxomer, polyethylene glycol and mixtures thereof.

Disintegrants according to the present invention include, but not limited to, microcrystalline cellulose, croscarmellose sodium, xylitol, polyplasdone (1-ethenylpyrrolidin-2-one), crospovidone, hydroxypropyl cellulose, low-substituted hydroxypropyl cellulose (L-HPC) and sodium starch glycolate or mixtures thereof.

Glidants according to the present invention include, but not limited to, silicon dioxide, magnesium trisilicate, starch, talc, colloidal silicon dioxide or silicon hydrogel or mixtures thereof.

Lubricants according to the present invention include, but not limited to, magnesium stearate, sodium stearyl fumarate, polyethylene glycol, stearic acid, metal stearates, boric acid, sodium chloride benzoate and acetate, sodium or magnesium lauryl sulfate or mixtures thereof.

Plasticizers according to the present invention include, but not limited to, triethyl citrate, triacetin, citric acid esters, phthalic acid esters, dibutyl sebacate, cetyl alcohol, polyethylene glycol, polysorbate or mixtures thereof.

Surfactants according to the present invention may include, but not limited to, dioctyl sulfosuccinate, polysorbates and polyoxyethylene alkyl esters and ethers thereof, glyceryl monolaurate saponins, sorbitan laurate, sodium lauryl sulfate, magnesium lauryl sulfate or mixtures thereof.

Preservatives according to the present invention include, but not limited to, methyl paraben, propyl paraben and salts thereof (e.g. sodium or potassium salts), sodium benzoate, citric acid, benzoic acid, butylated hydroxytoluene and butylated hydroxyanisole or mixtures thereof.

The present invention, in a preferred embodiment thereof, also include at least one or a mixture in the suitable amount of polyvinyl alcohol-polyethylene glycol copolymer, polyoxyethylene-polyoxypropylene block copolymer, stearyl macrogol glyceride, polyethylene glycol, povidone, cationic methacrylate, copovidone, derivatives of methacrylic acid copolymer, cellulose acetate phthalate, acetylated monoglyceride, dibutyl tartrate, diethyl phthalate, dimethyl phthalate, glycerine, propylene glycol, stearic acid, triacetin, triacetin citrate and tripropionin.

Excipients comprised within the content of the multi-layer tablet according to the present invention is selected and formulated so as to optimize disintegration rate of said tablet. In the formulation according to the present invention, disintegrant agent provided in each layer containing the active ingredients is in the range of 0.5% to 30% by weight of the layer. With disintegrant agent present in such amount, formulation gains the hardness required for the sandwich form as well as disintegrates quickly.

According to another embodiment of the present invention, NSAID and diacerein layers comprise croscarmellose sodium and hydroxypropyl cellulose as disintegrant. Said disintegrant agents swell by absorbing the water upon contact therewith and provide rapid disintegration of the tablet. It has been found out that the first and second layers comprising active ingredient provided in the multi-layer tablet formulation according to the present invention with croscarmellose sodium agent about 3% to 7% by weight of the layer and hydroxypropyl cellulose agent about 2% to 10% by weight of the layer causes synergistic effect on the disintegration time. Higher amounts can lead to adverse effects on the mechanical resistance of the formula while lower amounts can worsen the disintegration time. In said ranges, layers containing croscarmellose sodium and hydroxypropyl cellulose disintegrate rapidly as well as they are hard enough to take the multi-layer tablet form according to the present invention.

According to another embodiment, the present invention concerns inclusion of glidant in the layers of the multi-layer tablet formulation comprising NSAID and diacerein wherein said glidant is preferably colloidal silicon dioxide. Colloidal silicon dioxide provided in the layers is in the range of approximately 0.01% to 1% by weight of the layers. It has been found out that the effectiveness of the disintegrant agents increase with the colloidal silicon dioxide provided in said range in each layer. Colloidal silicon dioxide is a hydrophobic substance. Thus, large amount of said substance being present delays the contact of the disintegrant agents with water and leads to delayed disintegration of the tablet.

According to another embodiment, the present invention concerns multi-layer tablet formulation comprising NSAID and diacerein wherein lactose and derivatives thereof are comprised therein as excipient. Total lactose amount provided in the tablet according to the present invention is in the range of 14% to 43% by weight of total tablet weight. Thus, potential side effects that may be observed in the patient (e.g., lactose intolerance) are reduced. When NSAID and diacerein preparations available in the prior art are used separately, total lactose intake of the patient increases. When these two molecules are brought together in the form of multi-layer tablet, lactose intake decreases in the range of approximately 30% to 60% and thus, potential side effects that can be observed in the patient reduces.

According to another embodiment, in the multi-layer tablet formulation according to the present invention, lactose amount comprised in the layer containing NSAID is in the range of 7% to 43% by weight of respective layer and lactose amount comprised in the layer containing diacerein is in the range of 50% to 60% by weight of respective layer. With lactose being present in said ranges, multi-layer tablet is not affected by environmental conditions such as humidity in particular and becomes more stable. In addition, compressibility property of the tablet is at the optimum level with lactose provided in such ranges.

Pharmaceutically acceptable lactose according to the present invention is selected from the group comprising spray-dried lactose, beta-lactose, alpha lactose monohydrate, lactose anhydride or mixtures thereof, preferably, said lactose is spray-dried lactose.

The multi-layer tablet according to the present invention comprises pharmaceutically acceptable lactose having 98% of particles thereof with size in the range of approximately 300 µm to 350 µm.

From another perspective, the amount of NSAID group molecules provided in the multi-layer tablet formulation according to the present invention is in the range of 5mg to 1000mg and preferably, among said molecules, flurbiprofen is provided in the range of approximately 50mg to 300mg and dexketoprofen is provided in the range of approximately 5mg to 100mg and more preferably, flurbiprofen amount is 100mg or 50 mg and dexketoprofen amount is 25mg.

From another perspective, the amount of diacerein provided in the multi-layer tablet formulation according to the present invention is in the range of approximately 10mg to 100mg and preferably, diacerein amount is 50mg.

The present invention concerns a multi-layer tablet comprising NSAID layer wherein it includes, but may not be limited to, the following substances by weight of the respective layer:
a) approximately 5% to 60% by weight of NSAID,
b) approximately 5% to 60% by weight of diluent,
c) approximately 0.5% to 30% by weight of disintegrant,
d) approximately 2% to 10% by weight of binder,
e) approximately 0.1 % to 10% by weight of glidant,
f) approximately 0.1% to 10% by weight of lubricant.

The present invention concerns a multi-layer tablet comprising diacerein layer wherein it includes, but may not be limited to, the following substances by weight of the respective layer:
a) approximately 5% to 50% by weight of diacerein,
b) approximately 30% to 75% by weight of diluent,
c) approximately 0.5% to 30% by weight of disintegrant.
d) approximately 1% to 10% by weight of binder,
e) approximately 0.01% to 5% by weight of glidant,
f) approximately 1% to 10% by weight of lubricant.

From another perspective, the present invention concerns a multi-layer tablet in the form of sandwich comprising a barrier layer separating a first layer containing NSAID and a second layer containing diacerein wherein the barrier layer constitutes the middle layer of the multi-layer tablet and comprises pharmaceutically suitable excipients. Incompatibility of NSAID and diacerein molecules within the formulation is minimized or prevented by means of said barrier layer.

The pharmaceutically acceptable excipients provided in the barrier layer according to the present invention include binders, disintegrants, glidants, lubricants, plasticizers, surfactants, preservatives or mixtures thereof.

Multi-layer tablet formulation according to the present invention can be prepared by various conventional methods known in the respective technical field. Said methods can be given as, but not limited to, wet granulation, dry granulation, direct compression, melt granulation and double compression.

NSAID containing layer provided in the multi-layer tablet formulation according to the present invention can be prepared by various conventional methods known in the respective technical field wherein it is preferably prepared by wet granulation or dry granulation methods. Thus, potential flowability problems that can be encountered with NSAID molecules are resolved.

Diacerein containing layer provided in the multi-layer tablet formulation according to the present invention can be prepared by various conventional methods known in the respective technical field wherein it is preferably prepared by dry granulation or direct compression methods.

Barrier layer provided in the multi-layer tablet formulation according to the present invention can be prepared by various conventional methods known in the respective technical field wherein it is preferably prepared by direct compression method.

Finally, 3 layers obtained by methods according to the present invention are compressed together so as to form a tablet where the barrier layer is positioned in the middle.

The term formulation may refer to formulation as well as both the packaging or blisters in which the formulation is stored. Multi-layer tablet formulation according to the present invention is preferably stored in opaque PVC/PVDC packaging and the formulation thus becomes more stable. The main reason for this is the light sensitivity of the diacerein molecule.

### Examples

The following examples show the preferred oral pharmaceutical formulations of the present invention.

### Example 1:

Multi-layer tablet formulation, manufacturing method of which is described below, contains 100mg flurbiprofen and 50mg diacerein.

| | **Constituent** | **Amount per tablet (mg)** |
|---|---|---|
| **Layer I** | Flurbiprofen | 100.00 |
| | Lactose | 32.60 |
| | Microcrystalline cellulose | 132.00 |
| | Croscarmellose sodium | 14.00 |
| | Hydroxypropyl cellulose | 18.50 |
| | Colloidal silicon dioxide | 1.90 |
| | Magnesium stearate | 1.00 |
| | **First layer total** | **300.00** |
| **Intermediate Layer** | Sodium carboxymethylcellulose | 44.70 |
| | Polyvinylpyrrolidone | 6.00 |
| | Microcrystalline cellulose | 22.1 |
| | Yellow iron oxide | 0.30 |
| | Colloidal silicon dioxide | 0.40 |
| | Magnesium stearate | 1.5 |
| | **Intermediate layer total** | **75.00** |
| **Layer II** | Diacerein | 50.00 |
| | Lactose | 83.40 |
| | Croscarmellose sodium | 7.50 |
| | Hydroxypropyl cellulose | 5.00 |
| | Colloidal silicon dioxide | 0.10 |
| | Sodium stearyl fumarate | 4.00 |
| | **Second layer total** | **150.00** |

### Manufacturing method:

a) Preparation of the first layer containing flurbiprofen is carried out as follows:
   i. Flurbiprofen, a portion of microcrystalline cellulose, lactose, croscarmellose sodium and hydroxypropyl cellulose are provided;
   ii. Colloidal silicon dioxide and rest of the microcrystalline cellulose are sieved, added into the mixture obtained in step i. and mixed;
   iii. Tablets are compressed into bricks, crushed and sieved;
   iv. Magnesium stearate is added into the mixture obtained in step iii and mixed.
b) Preparation of the second layer containing diacerein is carried out as follows:
   i. Diacerein, a portion of lactose, croscarmellose sodium and hydroxypropyl cellulose are provided;
   ii. Colloidal silicon dioxide and rest of the lactose are sieved, added into the mixture obtained in step i and mixed;
   iii. Sodium stearyl fumarate is added into the mixture obtained in step ii and mixed.
c) Preparation of the barrier layer is carried out as follows:
   i. Sodium carboxymethyl cellulose, polyvinylpyrrolidone, a portion of microcrystalline cellulose and yellow iron oxide are provided;
   ii. Rest of the microcrystalline cellulose and colloidal silicon dioxide are sieved, added into the mixture obtained in step i and mixed;
   iii. Magnesium stearate is added into the mixture obtained in step ii and mixed.
d) Homogeneous mixtures obtained in steps a, b, and c are compressed so as to obtain a tablet in the form of sandwich with the barrier layer provided in the middle thereof.

### Example 2:

Multi-layer tablet formulation, manufacturing method of which is described below, contains 100mg flurbiprofen and 50mg diacerein.

| | **Constituent** | **Amount per tablet (mg)** |
|---|---|---|
| **Layer I** | Flurbiprofen | 100.00 |
| | Lactose | 124.50 |
| | Microcrystalline cellulose | 55.00 |
| | Croscarmellose sodium | 7.30 |
| | Hydroxypropyl cellulose | 9.80 |
| | Colloidal silicon dioxide | 2.10 |
| | Magnesium stearate | 1.30 |
| | **First layer total** | **300.00** |
| **Layer II** | Diacerein | 50.00 |
| | Lactose | 83.40 |
| | Croscarmellose sodium | 7,50 |
| | Hydroxypropyl cellulose | 5.00 |
| | Colloidal silicon dioxide | 0.10 |
| | Sodium Stearyl fumarate | 4.00 |
| | **Intermediate layer total** | **150.00** |
| **Intermediate Layer** | Carboxymethyl cellulose | 44.70 |
| | Polyvinil pyrollidone | 6.00 |
| | Microcrystalline cellulose | 22.1 |
| | Yellow Iron Oxide | 0.30 |
| | Colloidal silicon dioxide | 0.40 |
| | Magnesium Stearate | 1.5 |
| | **Second layer total** | **75.00** |

### Manufacturing method:

a) Preparation of the first layer containing flurbiprofen is carried out as follows:
   i. Flurbiprofen, microcrystalline cellulose, lactose and a portion of the hydroxypropyl cellulose are picked up and loaded into the fluidized bed dryer;
   ii. An aqueous solution is prepared with the rest of the hydroxypropyl cellulose;
   iii. Mixture obtained in step i. in the fluidized bed dryer is granulated with said solution, dried and milled;
   iv. Mixture obtained in step iii is transferred into the container;
   v. Croscarmellose sodium and colloidal silicon dioxide are added into the same container and mixed;
   vi. Magnesium stearate is added into the mixture obtained in step v and mixed.
b) Preparation of the second layer containing diacerein is carried out as follows:
   i. Diacerein, a portion of lactose, croscarmellose sodium and hydroxypropyl cellulose are mixed;
   ii. Colloidal silicon dioxide and rest of the lactose are sieved, added into the mixture obtained in step i and mixed;
   iii. Sodium stearyl fumarate is added into the mixture obtained in step ii and mixed.
c) Preparation of the barrier layer is carried out as follows:
   i. Sodium carboxymethyl cellulose, polyvinylpyrrolidone, a portion of microcrystalline cellulose and yellow iron oxide are mixed;
   ii. Rest of the microcrystalline cellulose and colloidal silicon dioxide are sieved, added into the mixture obtained in step i and mixed;
   iii. Magnesium stearate is added into the mixture obtained in step ii and mixed.
d) Homogeneous mixtures obtained in steps a, b, and c are compressed so as to obtain a tablet in the form of sandwich with the barrier layer provided in the middle thereof.

### Example 3: Multi-layer tablet formulation containing dexketoprofen and diacerein

| **Constituent** | **Amount per tablet (mg)** |
|---|---|
| Dexketoprofen trometamol (equivalent to 25mg dexketoprofen) | 36.91 |
| Lactose monohydrate | 138.6 |
| Corn starch | 65.0 |
| Sodium starch glycolate | 13.0 |
| Sodium stearyl fumarate | 6.5 |
| **First layer total** | **260.00** |
| Sodium carboxymethylcellulose | 44.70 |
| Polyvinylpyrrolidone | 6.00 |
| Microcrystalline cellulose | 22.1 |
| Yellow iron oxide | 0.30 |
| Colloidal silicon dioxide | 0.40 |
| Magnesium stearate | 1.5 |
| **Intermediate layer total** | **75.00** |
| Diacerein | 50.00 |
| Lactose | 83.40 |
| Croscarmellose sodium | 7.50 |
| Hydroxypropyl cellulose | 5.00 |
| Colloidal silicon dioxide | 0.10 |
| Sodium stearyl fumarate | 4.00 |
| **Second layer total** | **150.00** |

### Manufacturing method:

a) Preparation of the first layer containing dexketoprofen is carried out as follows:
   i. Dexketoprofen, lactose monohydrate and corn starch are picked up and loaded into the fluidized bed dryer;
   ii. Mixture obtained in step i in the fluidized bed dryer is granulated with water, dried and milled;
   iii. Mixture obtained in step ii is transferred into the container;
   iv. Sodium starch glycolate is added into the same contained and mixed;
   v. Sodium stearyl fumarate is added into the mixture obtained in step iv and mixed.
b) Preparation of the second layer containing diacerein is carried out as follows:
   i. Diacerein, a portion of lactose, croscarmellose sodium and hydroxypropyl cellulose are mixed;
   ii. Colloidal silicon dioxide and rest of the lactose are sieved, added into the mixture obtained in step i and mixed;
   iii. Sodium stearyl fumarate is added into the mixture obtained in step ii and mixed.
c) Preparation of the barrier layer is carried out as follows:
   i. Sodium carboxymethyl cellulose, polyvinylpyrrolidone, a portion of microcrystalline cellulose and yellow iron oxide are mixed;
   ii. Rest of the microcrystalline cellulose and colloidal silicon dioxide are sieved, added into the mixture obtained in step i and mixed;
   iii. Magnesium stearate is added into the mixture obtained in step ii and mixed.
d) Homogeneous mixtures obtained in steps a, b, and c are compressed so as to obtain a tablet in the form of sandwich with the barrier layer provided in the middle thereof.

## Claims

1. A solid oral multi-layer tablet in the form of sandwich, wherein it comprises:
i) a first layer containing a propionic acid derivative NSAID molecule as the first active agent and pharmaceutically acceptable excipient or excipients;
ii) a second layer containing diacerein molecule as the second active agent and pharmaceutically acceptable excipient or excipients; and
iii) a barrier layer positioned between these two layers and comprising pharmaceutically acceptable excipient or excipients.

2. A solid oral multi-layer tablet in the form of sandwich according to claim 1, wherein the pharmaceutically acceptable excipient provided therein comprises at least one of the binders, disintegrants, glidants, lubricants, plasticizers, surfactants, preservatives or mixtures thereof in the suitable amount.

3. A solid oral multi-layer tablet in the form of sandwich according to claim 2, wherein disintegrants are selected among microcrystalline cellulose, croscarmellose sodium, xylitol, polyplasdone (1-ethenylpyrrolidin-2-one), crospovidone, hydroxypropyl cellulose, low-substituted hydroxypropyl cellulose (L-HPC) and sodium starch glycolate or mixtures thereof.

4. A solid oral multi-layer tablet in the form of sandwich according to claim 3, wherein disintegrants are microcrystalline cellulose and croscarmellose sodium.

5. A solid oral multi-layer tablet in the form of sandwich according to any preceding claim, wherein the first and second layers comprise croscarmellose sodium agent in the range of 3% to 7% and hydroxypropyl cellulose agent in the range of 2% to 10% by weight of the layers.

6. A solid oral multi-layer tablet in the form of sandwich according to claim 2, wherein glidants comprised therein are selected among silicon dioxide, magnesium trisilicate, starch, talc, colloidal silicon dioxide or silicon hydrogel or mixtures thereof.

7. A solid oral multi-layer tablet in the form of sandwich according to claim 6, wherein glidant comprised therein is colloidal silicon dioxide.

8. A solid oral multi-layer tablet in the form of sandwich according to claim 7, wherein each layer comprises colloidal silicon dioxide in the range of 0.01% to 1% by weight of respective layers.

9. A solid oral multi-layer tablet in the form of sandwich according to claim 2, wherein binders comprised therein are selected from lactose, polymethacrylate, polyvinylpyrrolidone (povidone), hydroxypropyl methyl cellulose (HPMC), hydroxypropyl cellulose (HPC), carboxymethyl cellulose (CMC), methyl cellulose (MC), hydroxy ethyl cellulose, sodium carboxy methyl cellulose (NaCMC), carboxymethyl cellulose calcium, ethyl cellulose, polyethylene oxide, gelatin, starch, pregelatinized starch, xanthan gum, guar gum, alginate, carrageenan, pectin, carbomer, cellulose acetate phthalate, hydroxy propyl starch, polaxomer, polyethylene glycol and mixtures thereof.

10. A solid oral multi-layer tablet in the form of sandwich according to claim 9, wherein binder comprised therein is lactose.

11. A solid oral multi-layer tablet in the form of sandwich according to claim 10, wherein amount of lactose comprised therein is in the range of 14% to 43% by weight of the total tablet.

12. A solid oral multi-layer tablet in the form of sandwich according to claims 9 to 11, wherein lactose comprised therein is spray-dried lactose.

13. A solid oral multi-layer tablet in the form of sandwich according to any preceding claim, wherein the propionic acid derivative NSAID is selected from ibuprofen, naproxen, ketoprofen, fenoprofen, benoxaprofen, suprofen, flurbiprofen, ibuproxam, alminoprofen, dexibuprofen, dexketoprofen, indoprofen and mixture thereof.

14. A solid oral multi-layer tablet in the form of sandwich according to claim 13, wherein the propionic acid derivative NSAID is selected from flurbiprofen, dexketoprofen and/or mixture thereof.

## Patentansprüche

1. Feste orale Multischicht-Tablette in der Form eines Sandwich, wobei sie umfasst:
i) eine erste Schicht, enthaltend ein Propionsäurederivat-NSAID-Molekül als das erste aktive Mittel und ein oder mehrere pharmazeutisch akzeptable Bindemittel;
ii) eine zweite Schicht, enthaltend Diacerein-Molekül als das zweite aktive Mittel und ein oder mehrere pharmazeutisch akzeptable Bindemittel;
iii) eine Barriereschicht, die zwischen diesen beiden Schichten positioniert ist und ein oder mehrere pharmazeutisch akzeptable Bindemittel umfasst.

2. Feste orale Multischicht-Tablette in der Form eines Sandwich nach Anspruch 1, wobei das darin bereitgestellte pharmazeutisch akzeptable Bindemittel wenigstens eines von Bindematerialien, Zersetzungsmitteln, Gleitmitteln, Schmiermitteln, Weichmachern, oberflächenaktiven Mitteln, Konservierungsmitteln oder Mischungen davon in der geeigneten Menge umfasst.

3. Feste orale Multischicht-Tablette in der Form eines Sandwich nach Anspruch 2, wobei die Zersetzungsmittel ausgewählt sind aus mikrokristalliner Cellulose, Croscarmellose-Natrium, Xylitol, Polyplasdone (1-Ethenylpyrrolidin-2-on), Crospovidone, Hydroxypropylcellulose, gering substituierte Hydroxypropylcellulose (L-HPC) und Natriumstärkeglykolat oder Mischungen davon.

4. Feste orale Multischicht-Tablette in der Form eines Sandwich nach Anspruch 3, wobei die Zersetzungsmittel mikrokristalline Cellulose und Croscarmellose-Natrium sind.

5. Feste orale Multischicht-Tablette in der Form eines Sandwich nach einem der vorangehenden Ansprüche, wobei die ersten und zweiten Schichten Croscarmellose-Natrium-Mittel im Bereich von 3 % bis 7 % und Hydroxypropylcellulose-Mittel im Bereich von 2 % bis 10 % umfassen, bezogen auf das Gewicht der Schichten.

6. Feste orale Multischicht-Tablette in der Form eines Sandwich nach Anspruch 2, wobei die darin enthaltenen Gleitmittel ausgewählt sind aus Siliziumdioxid, Magnesiumtrisilikat, Stärke, Talcum, kolloidalem Siliziumdioxid oder Siliziumhydrogel oder Mischungen davon.

7. Feste orale Multischicht-Tablette in der Form eines Sandwich nach Anspruch 6, wobei das darin enthaltene Gleitmittel kolloidales Siliziumdioxid ist.

8. Feste orale Multischicht-Tablette in der Form eines Sandwich nach Anspruch 7, wobei jede Schicht kolloidales Siliziumdioxid im Bereich von 0,01 % bis 1 % umfasst, bezogen auf das Gewicht der jeweiligen Schichten.

9. Feste orale Multischicht-Tablette in der Form eines Sandwich nach Anspruch 2, wobei die darin enthaltenen Bindematerialien ausgewählt sind aus Lactose, Polymethacrylat, Polyvinylpyrrolidon (Povidon), Hydroxypropylmethylcellulose (HPMC), Hydroxypropylcellulose (HPC), Carboxymethylcellulose (CMC), Methylcellulose (MC), Hydroxyethylcellulose, Natriumcarboxymethylcellulose (NaCMC), Carboxymethylcellulosecalcium, Ethylcellulose, Polyethylenoxid, Gelatine, Stärke, vorgelierte Stärke, Xanthan-Gum, Guar-Gum, Alginat, Carrageen, Pectin, Carbomer, Celluloseacetatphthalat, Hydroxypropylstärke, Polaxomer, Polyethylenglykol und Mischungen davon.

10. Feste orale Multischicht-Tablette in der Form eines Sandwich nach Anspruch 9, wobei das darin enthaltene Bindematerial Lactose ist.

11. Feste orale Multischicht-Tablette in der Form eines Sandwich nach Anspruch 10, wobei die darin enthaltene Menge an Lactose im Bereich von 14 % bis 43 % ist, bezogen auf das Gewicht der gesamten Tablette.

12. Feste orale Multischicht-Tablette in der Form eines Sandwich nach den Ansprüchen 9 bis 11, wobei die darin enthaltene Lactose sprühgetrocknete Lactose ist.

13. Feste orale Multischicht-Tablette in der Form eines Sandwich nach einem der vorangehenden Ansprüche, wobei das Propionsäurederivat-NSAID ausgewählt ist aus Ibuprofen, Naproxen, Ketoprofen, Fenoprofen, Benoxaprofen, Suprofen, Flurbiprofen, Ibuproxam, Alminoprofen, Dexibuprofen, Dexketoprofen, Indoprofen und einer Mischung davon.

14. Feste orale Multischicht-Tablette in der Form eines Sandwich nach Anspruch 13, wobei das Propionsäurederivat-NSAID ausgewählt ist aus Flurbiprofen, Dexketoprofen und/ oder einer Mischung davon.

## Revendications

1. Comprimé oral solide multicouche en forme de sandwich, le comprimé comprenant :
i) une première couche contenant une molécule d'AINS dérivée de l'acide propionique comme premier agent actif et un ou plusieurs excipients pharmaceutiquement acceptables,
ii) une seconde couche contenant la molécule diacéréine comme second agent actif et un ou plusieurs excipients pharmaceutiquement acceptables, et
iii) une couche barrière positionnée entre ces deux couches et comprenant un ou plusieurs excipients pharmaceutiquement acceptables.

2. Comprimé oral solide multicouche en forme de sandwich suivant la revendication 1, dans lequel l'excipient pharmaceutiquement acceptable prévu comprend au moins un agent parmi le groupe comprenant les liants, les désintégrants, les agents de glissement, les lubrifiants, les plastifiants, les agents tensio-actifs, les conservateurs ou des mélanges de ceux-ci en quantité appropriée.

3. Comprimé oral solide multicouche en forme de sandwich suivant la revendication 2, dans lequel les agents désintégrants sont sélectionnés parmi le groupe comprenant la cellulose microcristalline, la croscarmellose sodique, le xylitol, le polyplasdone (1-éthénylpyrrolidine-2-one), la crospovidone, l'hydroxypropyl-cellulose, l'hydroxypropylcellulose à faible degré de substitution (L-HPC) et le glycolate sodique d'amidon ou des mélanges de ceux-ci.

4. Comprimé oral solide multicouche en forme de sandwich suivant la revendication 3, dans lequel les désintégrants sont la cellulose microcristalline et la croscarmellose sodique.

5. Comprimé oral solide multicouche en forme de sandwich suivant une quelconque des revendications précédentes, dans lequel la première et la seconde couche comprennent de 3% à 7% de croscarmellose sodique et de 2 à 10% de cellulose microcristalline par poids des couches comme agents désintégrants.

6. Comprimé oral solide multicouche en forme de sandwich suivant la revendication 2, dans lequel les agents de glissement contenus sont sélectionnés parmi le groupe comprenant le dioxyde de silicone, le trisilicate de magnésium, l'amidon, le talc, le dioxyde de silicone colloïdal ou l'hydrogel de silicone ou des mélanges de ceux-ci.

7. Comprimé oral solide multicouche en forme de sandwich suivant la revendication 6, dans lequel l'agent de glissement contenu est le silicone colloïdal.

8. Comprimé oral solide multicouche en forme de sandwich suivant la revendication 7, chaque couche comprenant de 0,01% à 1% de dioxyde de silicone colloïdal par poids des couches respectives.

9. Comprimé oral solide multicouche en forme de sandwich suivant la revendication 2, dans lequel les agents liants contenus sont sélectionnés parmi le groupe comprenant le lactose, le polyméthacrylate, le polyvinylpyrrolidone (povidone), l'hydroxypropylméthylcellulose (HPMC), l'hydroxypropylcellulose (HPC), la carboxyméthylcellulose (CMC), la méthylcellulose (MC), l'hydroxyéthylcellulose, la carboxyméthylcellulose sodique (NaCMC), la carboxyméthylcellulose calcique, l'éthylcellulose, l'oxyde de polyéthylène, la gélatine, l'amidon, l'amidon prégélatinisé, la gomme de xanthane, la gomme de guar, l'alginate, la carraghénane, la pectine, le carbomère, l'acétophtalate de cellulose, l'amidon d'hydroxypropyle, le poloxamère, le polyéthylène glycol et des mélanges de ceux-ci.

10. Comprimé oral solide multicouche en forme de sandwich suivant la revendication 9, dans lequel l'agent liant contenu est du lactose.

11. Comprimé oral solide multicouche en forme de sandwich suivant la revendication 10, dans lequel la quantité de lactose contenue se situe dans la plage de 14 à 43% par poids du comprimé total.

12. Comprimé oral solide multicouche en forme de sandwich suivant la revendication 11, dans lequel la lactose contenue est de la lactose séchée par pulvérisation.

13. Comprimé oral solide multicouche en forme de sandwich suivant une quelconque des revendications précédentes, dans lequel l'AINS dérivé de l'acide propionique est sélectionné parmi le groupe comprenant l'ibuprofène, le naproxène, le kétoprofène, le benoxaprofène, le suprofène, le flurbiprofène, l'ibuproxame, l'alminoprofène, le dexibuprofène, le dexcétoprofène, l'indoprofène et des mélanges de ceux-ci.

14. Comprimé oral solide multicouche en forme de sandwich suivant la revendication 13, dans lequel l'AINS dérivé de l'acide propionique est sélectionné parmi le groupe comprenant le flurbiprofène, le dexcétoprofène et ou des mélanges de ceux-ci.
